# EUROPEAN PATENT APPLICATION

(11) **EP 1 623 705 A1**
(43) Date of publication of application: **08.02.2006**
(21) Application number: 04291755.9
(22) Date of filing: 09.07.2004
(51) Int. Cl.: A61K 31/215, A61P 25/18

(54) **Use of phenylethanolaminotetralines for preparing anxiolytic drugs**

(71) Applicant: Sanofi-Aventis, 75013 Paris (FR)
(72) Inventor: Drago, Filippo, 95030 S. Agata Li Battiati(Catania) (IT); Consoli, Daniele, 95126 Catania (IT); Mazzola, Carmen, 95125 Catania (IT); Micale, Vincenzo, 97019 Vitoria (Ragusa) (IT); Griebel, Guy, 92260 Fontenay-aux-Roses (FR)
(74) Representative: Kugel, Dominique

(57) **Abstract**

Use of at least one phenylethanolaminotetraline of general formula (I): 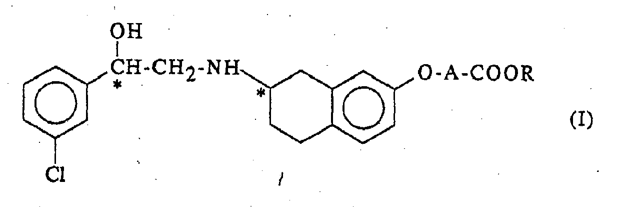 wherein A represents a (C₁-C₄)alkylene group, and R is hydrogen or a (C₁-C₄)alkyl group, or one of its pharmaceutically acceptable salts,
for the preparation of a drug for treating anxiety disorders.

## Description

The present invention concerns the use of some phenylethanolaminotetralines for preparing drugs, intended to the treatment of anxiety.

An object of the present invention is the use of at least one phenylethanolaminotetraline of general formula (I): 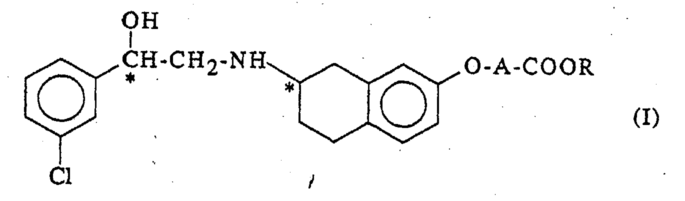
wherein:
A represents a (C₁-C₄)alkylene group, and
R is hydrogen or a (C₁-C₄)alkyl group,
or one of its pharmaceutically acceptable salts,
for the preparation of a drug for treating anxiety disorders.

Compounds of general formula (I) are useful for the preparation of a drug for treating anxiety disorders such as generalized anxiety, panic, acute and posttraumatic stress, social anxiety, and obsessive compulsive disorders.

Another object of the invention is a method for treating anxiety, which comprises administering to a mammal a therapeutically effective amount of at least one phenylethanolaminotetraline derivative of general formula (I).

The preparation and the characteristics of phenylethanolaminotetralines according to the invention, have been described in documents EP 0 211 721, EP 0 303 545, EP 0 303 546 and EP 0 626 367. Their preparation and their activity with respect to intestinal motricity were disclosed in EP 0 303 546. A new crystalline form (referred as II (or B) form) of ethyl [(7S) 7-[(2R) 2-(3-chlorophenyl)-2-hydroxyethylamino]-5,6,7,8-tetrahydronaphthalen-2-yloxy]-acetate hydrochloride (this compound was disclosed in EP 0 303 546, Example 12), has recently been found (WO 03/002510).
Additionally, it was shown that phenylethanolaminotetralines according to the invention were therapeutically interesting as antidepressant agents (EP 0 489 640, and Simiand et al, *Eur. J. Pharmacol.,* (1992), 219, p. 193-201).

It has now been found that the compounds of general formula (I) are therapeutically very interesting as anxiolytic agents.

The anxiolytic properties of the compounds of general formula (I) have been investigated by means of a set of tests in animals conventionally employed in pharmacology and generally considered predictive of anxiolytic activity in man (Griebel et al., *The Journal of Pharmacology and Experimental Therapeutics,* (2002), 301 (1), p. 333-345; Gipsen et al., (1975), *Life Sci.,* 17, p. 645-652; and Salomé et al., Sanofi-Synthelabo Research, CNS Department, Bagneux, France: presentation at the 5^{th} Congress on stress, London, UK, June 18-19, 2004, n°310). More particularly, compounds of general formula (I) have been tested in the following tests:
- the Novelty Induced Grooming Test in rats,
- the Elevated Plus Maze test in rats,
- the Ultrasonic Vocalization test in rats,
- the Punished Drinking test in rats,
- the Social Interaction test in gerbils,
- the Mouse Defense test Battery,

Possible behavioural or locomotor activity modifications following administration of compounds according to the invention have been evaluated. Compounds according to the invention were tested at a dosage comprised in the range of 0.3 to 50 mg/kg. Data were analyzed with one-way analysis of variance (ANOVA).
These tests illustrate anxiolytic-like effects of compounds according to the invention. However, the demonstration of anxiolytic-like effects is not limited to them only.

### 1) The Novelty Induced Grooming test

### (Gipsen et al., (1975), ibid.)

Male rats of the Wistar strain (purchased from Morini, Italy) weighing from 200 to 220 g were used throughout this test. All animals were gently manipulated by experienced facilities' handlers, avoiding any environmental or physical stress. Compounds according to the invention to be tested were injected intraperitoneally (i.p.) to a group of rats at a dosage 10 mg/kg. Control animals received i.p. the same volume of the vehicle (i.e, physiological saline). Rats received only one injection of a standard volume of 1 mL solution with a 23-gauge stainless steel needle of 31-millimetre length (drug or vehicle), 30 minutes prior to the behavioural procedures. The animals were randomly assigned to any treatment group and were used only once.
Novelty-induced grooming behaviour was observed between 15 and 18 hours, under the same environmental conditions according to the method described in Drago et al., (1980), *Eur. J. Pharmacol.,* 65, p. 457, 458.
Rats were placed individually into plexiglas boxes (24x12x24 cm) in a low noise room. After a minute of adaptation, the behaviour of the rats was sampled every 15 seconds, and the occurrence of grooming was recorded in a session of 30 minutes. The occurrence of the following single elements of grooming was scored as grooming: washing (i.e., vibrating movements of the fore paws in front of the snout and licking of the same paws leading to a series of strokes along the snout and semicircular movements over the top of the head), scratching (that is scratching of the body by one of the limbs), licking (that is licking of the body fur, limbs and tail), and genital grooming (that is licking of genital area). Grooming behaviour of all animals was recorded on a tape using a video-camera (Hitachi Videocam) and then replayed and scored by two independent observers. The mean score of the two observations was used for the statistical analysis.

### Results:

### Number of grooming episodes:

Control rats show a number of grooming episodes of 37 (± 3.61), whereas the number of grooming episodes for rats treated with a compound according to the present invention, ethyl [(7S) 7-[(2R) 2-(3-chlorophenyl)-2-hydroxyethylamino]-5,6,7,8-tetrahydronaphthalen-2yloxy]-acetate hydrochloride (named compound A in the present invention) at 10 mg/kg, is 17.71 (± 5.91).
Rats treated with compound A show a considerable decrease in grooming score, thus demonstrating that they better and more quickly adapt themselves to an environmental stress than control rats, thus exhibiting an anxiolytic-like profile.

### 2) The Elevated Plus Maze test

### (Pellow S. et al., Journal of Neuroscience Methods, (1985), 14(3), p.149-67)

The animals, their preparation and the i.p. dosage of compound to be tested are the same as those described in the Novelty Induced Grooming test.

The apparatus consisted of a plus-shaped maze, elevated of 50 centimetres from the floor, with two opposite open arms (50x10 cm) crossed at right angles by two arms of the same dimensions, but enclosed by 40-centimetre high walls with an open roof. In addition, a 1-centimetre high edge made of plexiglas surrounded the open arms to avoid falls. Illumination measured at the centre of the maze was 40 lux. After treatment, the animal was placed at the centre of the plus maze with its nose in the direction of one of the closed arms, and observed for 5 minutes, according to the following parameters: number of entries in the open and closed arms (this parameter can also be expressed by means of percentage of entries in the open arms), and time of presence in each of them (this parameter can also be expressed by means of percentage of time stayed in the open arms. The time of presence measures the time spent by the animal in the open and closed arms. The animals were divided into four groups with 7 animals per group.

### Results

The following parameters have been evaluated.

### Number of entries in the open arms:

Control rats show a mean number of 4 entries into the open arms, which represents 40% of total entries. Treatment with compound A at 10 mg/kg significantly increased the mean number (to 7) and the percentage (to 50 %) of entries into the open arms.

### Time spent by the animal in the open arms:

Control rats spent a mean time of 78 seconds in the open arms (32 % of the time). Both these parameters were significantly increased (to a mean of 98 seconds and 42% of the time) in rats treated with compound A at 10 mg/kg.

Compound A reduces the animal's aversion to the open arms and promotes the exploration thereof, thus exhibiting an anxiolytic-like effect.

### 3) The Ultrasonic Vocalizations (USVs) test

### (Molewijk et al., Psychopharmacology, (1996),128, p. 31-38)

The animals, their preparation and the i.p. dosage of compound to be tested are the same as those described in the Novelty Induced Grooming test.

This test consists of two different sessions: training and testing sessions. In the training procedure, all animals were trained for 2 days prior to testing. Training consisted of placing the animal in a standard operant chamber (ENV-018M, Med Associates, Georgia, VT) where shock could be delivered from a programmable shocker (Model ENV-413, Med Associates) and where it is equipped with an ultrasonic detector (Mini-3 Bat Detector, Ultra Sound Advice, UK). On each of two consecutive days, all the animals received 20 shocks (1 mA, 4 seconds) separated by a random interval that averaged 60 seconds and ranging from 30 to 90 seconds. Each shock was administered concurrently with a 4-Watt light and an acoustic tone (85 dB, 4 s). The chamber was dark between each shock presentation.
On the day following training, animals were administered 10 mg/kg of compound A and placed in the operant apparatus for testing. This test consisted of 20 individual 4-second presentations of the acoustic tone and the 4-Watt light presented according to a random interval that averaged 60 seconds (30-90 seconds). USVs (18-22 KHz) were recorded over the intertrial interval and were expressed as a sum of total time spent vocalizing for each animal. No shocks were administered on the test day.

### Results

### Conditioned USVs:

Control rats show conditioned USVs of more than 210 seconds duration. In comparison, rats treated with compound A at 10 mg/kg exhibited fewer than 120 seconds of USVs, i.e. an almost 50% decrease.
Thus, compound A reduces the amount of vocalizations in the ultrasonic range, which is characteristic of an anxiolytic-like effect.

### 4) The Punished Drinking test

### (Griebel et al., (2002), ibid.)

Male Sprague-Dawley and Wistar rats (Iffa Credo, L'Arbresle, and Charles River, Saint-Aubin-lès-Elbeuf, France), weighing 180 to 250 g were used. Experiments were performed 30 minutes after i.p. injection of 10 mg/kg of the drug or vehicle.

The procedure was a modification of the technique described by Vogel et al., *Psychopharmacologia,* (1971), 21, p. 1-7. At the beginning of the experiment, rats, deprived of water but not of food for 48 hours prior to testing, were placed in cages (32 x 25 x 30 cm) with a stainless steel grid floor. Each cage was placed in sound-attenuated boxes that were well ventilated and contained a drinking tube connected to an external 50 mL burette filled with tap water. Trials were started only after the animal's tongue entered in contact with the drinking tube for the first time. An electric shock (0.6 mA/500 ms) was delivered to the tongue after every twenty licks. The number of shocks was recorded automatically during a 5-minute period. Subsequent comparisons between treatment groups and control were carried out using Dunnett's *t*-test.

### Results:

### Number of shocks:

The number of shocks is 10 for control rats, whereas the administration of compound A at 10 mg/kg significantly increased punished responding to 30 shocks.
Compound A increases the rate of responding suppressed by punishment, thus exhibiting an anxiolytic-like activity.

### 5) The Social Interaction test

### (Salomé et al., Sanofi-Synthelabo Research, CNS Department, Bagneux, France, ibid.)

Male Mongolian gerbils (7-week old, 50-60 g, CERJ, France) were used. Experiments were performed 1 hour after *per os* (p.o.) administration of 10 mg/kg of the drug or vehicle.
The procedure was based on that described initially by File et al., *Brain Res.,* (2001), 888, p. 311-313. Testing lasting two days. On day 1, gerbils were placed individually in a plastic box (30×30×20 cm) under high light conditions (300 lux) for a 10-minute free-exploration period to familiarize with the apparatus. Twenty-four hours later, two gerbils of the same weight but unfamiliar to each other were placed together in the box they had previously explored for a 4 minute 30 seconds observation period. Interaction time (sniffing, chasing, grooming the partner) was recorded manually for each pair of gerbils. Locomotor activity (distance travelled in centimetres) was measured with an automated tracking system (Ethovision, Noldus).

### Results:

### Social interaction time:

Control Gerbils show an interaction time of 30 seconds, whereas gerbils treated with compound A at 10 mg/kg exhibit significant increases in social interaction up to 70 seconds.
Compound A produces significant increases of social interaction, thus exhibiting an anxiolytic-like activity.

### 6) The Mouse Defense test Battery

### (Griebel et al., (2002), ibid.)

Male OF1 mice weighing 17 to 32 g were supplied by Charles River, Iffa Credo or Janvier (Le Genest, France). Experiments were performed 1 hour after p.o. administration of 0.3 mg/kg or 3 mg/kg of the drug or vehicle.

The test was conducted in an oval runway as described (Griebel et al., 1997), 23, p. 19-31). Tested mice were submitted to a 3-minute familiarization period sixty minutes after p.o. administration of compound A.

### (a) The rat avoidance test / flight:

Immediately after the 3-minute familiarization period, the experimenter introduced a hand-held dead rat (killed by CO₂ inhalation just before the beginning of the experiment) five times at one end of the runway. The following parameter was recorded: avoidance distance (cm).

### (b) Risk Assessment/Chase test:

The rat was then brought up to the subject at a speed of approximately 2 m/s. A constant distance of 2 meters separated the rat and the subject when the former was introduced in the runway. The following parameter was recorded: number of stops (pauses in movement). The rat was removed after the chase was completed.

### (c) Defensive Aggression/Straight alley:

By the closing of two doors (60 cm distant from each other), the runway was then converted to a straight alley in which the subject was constrained. The experimenter brought the rat up to contact the subject in the straight alley. Approaches were directed quickly (within 1 second) to the subject's head. For each such contact, bites by the subjects were recorded.

### Results:

### (a) Avoidance distance:

Control mice show an avoidance distance of 120 cm, compared to mice treated with compound A at 3 mg/kg, which show an avoidance distance of 75 cm.
Compounds A produces a decrease of the avoidance distance, thus demonstrating an anxiolytic-like effect.

### (b) Number of stops

Control mice show 9 stops, compared to mice treated with compound A at 3 mg/kg, which show 6 stops.
Compounds A produces a decrease of the number of stops, thus demonstrating an anxiolytic-like effect.

### (c) Number of bites:

Control mice bite the rat about 2.75 times. In comparison, mice treated with compound A at 0.3 mg/kg, bite the rat about 1.3 times.
Compound A produces a decrease of the number of defensive bites, which is characteristic of an anxiolytic-like activity.

Another object of the present invention is a method of treatment of anxiety which comprises administering to a mammal an anxiolytic effective dose of at least one compound of formula (I) or a pharmaceutically acceptable salt thereof.

As used herein, the term pharmaceutically acceptable salts includes the acid addition salts of pharmaceutically acceptable mineral or organic acids as well as the salts of the compounds of formula (I) wherein R is hydrogen with mineral bases, preferably those with alkali metals such as sodium or potassium, or with pharmaceutically acceptable organic bases.
Among the salts of the compounds of general formula (I), there may be cited hydrochloric acid, hydrobromic acid, acetic acid, formic acid, propionic acid, oxalic acid, fumaric acid, maleic acid, succinic acid, benzoic acid, cinnamic acid, mandelic acid, citric acid, malic acid, tartaric acid, aspartic acid, glutamic acid, methanesulfonic acid, benzenesulfonic acid, p-toluenesulfonic acid, and the like.
The hydrochloride is a preferred acid addition salt used according to the present invention.
In the above formula (I), the two asymmetric carbons are marked by an asterisk. All the compounds of formula (I) may therefore exist in at least four isomeric forms [(R,R), (R,S), (S,S), and (S,R)].
The optically pure isomers, as well any mixture of two, three, or all four isomers, in any proportion, may be employed according to the present invention.
An additional chiral center may be present in the radical A. Analogously, the stereoisomers derived from such additional chiral center and their mixtures may be employed according to the present invention. It is however understood that, as often happens when dealing with pharmaceutically active compounds possessing one or more chiral centers, the different stereoisomers may have different activity levels. If desired, the person skilled in the art may, on the basis of the indications given in the present application and his own experience, choose from the different stereoisomers of formula (I) the compound or compounds which are therapeutically most interesting.
A crystalline form of a phenylethanolamine, ethyl [(7S) 7-[(2R) 2-(3-chlorophenyl)-2-hydroxyethylamino]-5,6,7,8-tetrahydronaphthalen-2-yloxy]-acetate hydrochloride may be employed according to the present invention. In a particularly preferred embodiment, crystalline II form (or B form) of ethyl [(7S) 7-[(2R) 2-(3-chlorophenyl)-2-hydroxyethylamino]-5,6,7,8-tetrahydronaphthalen-2-yloxy]-acetate hydrochloride may be used.

For the treatment of anxiety, the compounds of general formula I may be administered orally, sublingually, transdermally, rectally, subcutaneously, intramuscularly, or intravenously.
The amount of active principle to be administered will depend, as usual, on the nature and severity of the pathological conditions to be treated as well as on the weight of the patient and the administration route.

In human beings the daily dosage typically varies between 0.1 and 30 mg/kg of body weight, and preferably between 1 and 10 mg/kg of body weight. Said daily dosage is generally subdivided in 2, 3, or 4 administrations.
Preferably, for the treatment of anxiety, the active principles (the compounds of general formula (I) and their pharmaceutically acceptable salts) are formulated in oral unit dosage forms containing from 10 to 800 mg and more preferably from 100 to 400 mg of active principle in admixture with a pharmaceutical vehicle. Unit dosage forms suitable for oral administration include tablets, capsules, powders, granules, and solutions or suspensions for oral use.
When a solid composition is prepared in the form of tablets, the main active ingredient is mixed with a pharmaceutical carrier such as gelatine, starch, lactose, magnesium stearate, talc, arabic gum, and the like. The tablets may be coated with sucrose or other appropriate materials or they may be treated so that their activity is extended or delayed and they continuously release a predetermined amount of active ingredient.
A preparation in the form of capsules is obtained by mixing the active ingredient with a diluent and filling the obtained mixture in soft or hard capsules. A preparation in the form of a syrup or elixir or for the administration in drops, may contain the active ingredient jointly with a sweetening agent, possibly acaloric, methylparaben and propylparaben as antiseptics, a flavoring agent and an appropriate dye. Water-dispersible powders or granules may contain the active principle mixed with dispersing agents, wetting agents or suspending agents such as polyvinylpyrrolidone and the like, and with sweetening or flavouring agents as well.
For rectal administration, suppositories can be employed which are prepared with vehicles melting at rectal temperature, such as cocoa butter or polyethyleneglycols.
For sublingual administration microtablets or microcapsules can be prepared which, placed under the tongue, will rapidly dissolve. These compositions will generally contain the active ingredient in admixture with wetting and/or dispersing agents and optionally with absorption enhancers.
For transdermal administration, the use of polymeric diffusion matrices for the continuous and preferably sustained release of the active principle can be devised as well as the use of the active principle as a microemulsion with excipients adapted for contact with the skin.
For parenteral administration, aqueous suspensions, isotonic saline solutions, or sterile injectable solutions are used which contain pharmaceutically compatible dispersing and/or wetting agents, for example propylene glycol or butyleneglycol.
The active principle of formula (I) may also be formulated in the form of microcapsules, possibly with one or more carriers or additives.
The active principle of general formula (I) may be administered as the free base or a pharmaceutically acceptable salt thereof, as such or complexed with a cyclodextrine, or even in admixture with or associated to other active principles.

## Claims

1. Use of at least one compound of general formula (I): 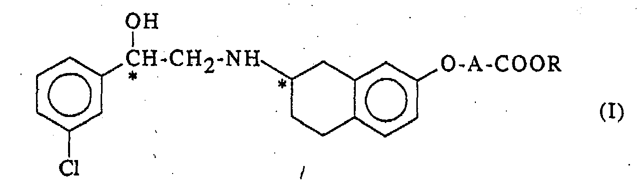
wherein:
A represents a (C₁-C₄)alkylene group, and
R stands for a hydrogen atom or a (C₁-C₄)alkyl group,
or one of its pharmaceutically acceptable salts,
for the preparation of a drug for treating anxiety.

2. Use according to claim 1, in which A represents a methylene or isopropylidene group.

3. Use according to any one of claims 1 or 2, **characterized in that** the carbon atom linked to the hydroxy group has absolute configuration (R).

4. Use according to any one of claim 1 to 3, **characterized in that** R is methyl or ethyl.

5. Use according to claim 4, **characterized in that** the compound of formula (I) is ethyl [(7S) 7-[(2R) 2-(3-chlorophenyl)-2-hydroxyethylamino]-5,6,7,8-tetrahydronaphthalen-2yloxy]-acetate hydrochloride.

6. Use according to claim 5, **characterized in that** the compound of formula (I) is ethyl [(7S) 7-[(2R) 2-(3-chlorophenyl)-2-hydroxyethylamino]-5,6,7,8-tetrahydronaphthalen-2yloxy]-acetate hydrochloride in crystalline II form.

7. A method for treating or preventing anxiety, which comprises administering to a mammal a therapeutically effective daily dosage of at least one phenylethanolaminotetraline derivative of general formula (I) or one of its pharmaceutically acceptable salts.

8. A method according to claim 7, wherein said effective daily dosage is comprised between 0.1 to 30 mg/kg body weight.

9. A method according to any one of claims 7 or 8, wherein said effective daily dosage is comprised between 1 to 10 mg/kg.
